# EUROPEAN PATENT APPLICATION

(11) **EP 4 458 860 A1**
(43) Date of publication of application: **06.11.2024**
(21) Application number: 22916691.3
(22) Date of filing: 27.12.2022
(51) Int. Cl.: C07K 16/40, A61P 25/28, A61P 25/24, G01N 33/68, A61K 39/00

(54) **ANTIBODY SPECIFICALLY BINDING TO ASM, AND USE THEREOF**

(30) Priority: 27.12.2021 KR 20210187953
(71) Applicant: Kyungpook National University Industry-Academic Cooperation Foundation, Daegu 41566 (KR)
(72) Inventor: BAE, Jae-Sung, Daegu 41566 (KR); JIN, Hee Kyung, Daegu 41566 (KR); PARK, Min Hee, Daegu 41566 (KR); CHOI, Byung Jo, Daegu 41566 (KR)
(74) Representative: JWP Patent & Trademark Attorneys
(86) International application number: PCT/KR2022/021379
(87) International publication number: WO 2023/128550

(57) **Abstract**

The present invention relates to an antibody that specifically binding to acid sphingomyelinase (ASM) and a use thereof and, more specifically to an anti-ASM monoclonal antibody which has very high binding sensitivity and specificity to ASM protein and an excellent inhibitory effect on ASM activity, and to a use thereof in treatment/diagnosis of neurodegenerative diseases.

## Description

### TECHNICAL FIELD

The present application claims priority to Korean Patent Application No. 2021-0187953 filed on December 27, 2021, and the entire specification thereof is incorporated herein by reference.

The present invention relates to an antibody that specifically binds to acid sphingomyelinase (ASM) and use thereof and, more specifically to an anti-ASM monoclonal antibody which has very high binding sensitivity and specificity for an ASM protein and is very effective in inhibiting the activities of ASM, and use thereof in treating or diagnosing neurodegenerative diseases.

### BACKGROUND ART

Sphingolipid metabolism regulates normal cell signaling, and abnormal changes in sphingolipid metabolism affect various neurodegenerative diseases comprising Alzheimer's disease. Meanwhile, acid sphingomyelinase (ASM), an enzyme that regulates sphingolipid metabolism, is a protein expressed in almost all types of cells and plays an important role in sphingolipid metabolism and cell membrane turnover.

The expression of ASM is significantly increased in the brains of patients with neurodegenerative diseases comprising Alzheimer's disease, as compared to normal people. Further, it has been reported that inhibiting the expression of overexpressed ASM or inhibiting the activities of ASM may inhibit the accumulation of amyloid-β (Aβ) and improve learning and memory to produce the effect of treating degenerative neurological diseases (Korean Patent No. 1521117). In addition, it has recently been reported that the activities of ASM have increased in neurological diseases such as depression, and that inhibition of ASM has the effect of alleviating depression (Nature Medicine (2013), 19(7):934-938; and PLoS One (2016), 11(9): e0162498). Therefore, the development of ASM inhibitors, that is, substances that can inhibit the expression or activities of ASM, is promising as a useful method for treating various diseases caused by an increase in ASM, which comprise neurodegenerative diseases and depression.

Meanwhile, although no direct inhibitor of ASM has been developed to date, several inhibitors that can indirectly inhibit ASM have been identified. First, tricyclic antidepressants (e.g., amitriptyline (AMI), desipramine, and imipramine), which are the most widely used indirect inhibitors of ASM, are actually used clinically as antidepressant drugs. Although such drugs were not initially developed as ASM inhibitors, various studies have proven that they exhibit ASM inhibitory effects. The main mode of action of tricyclic antidepressants is to increase neurotransmitter activities by inhibiting neurotransmitter reuptake in nerve cells, and the secondary mode of action is to inhibit ASM. However, tricyclic antidepressants may act on the nervous system and nerve cells and cause side effects such as dizziness, increased light sensitivity, and vomiting. Therefore, the development of new substances that can directly inhibit ASM activities is necessary.

### SUMMARY OF INVENTION

### TECHNICAL PROBLEM

The present inventors conducted extensive research to develop a new antibody which has excellent specificity and sensitivity for ASM to detect an ASM protein whose expression increases in patients with neurodegenerative diseases such as Alzheimer's disease, thereby diagnosing neurodegenerative diseases and directly inhibiting the activities of ASM to treat neurodegenerative diseases. As a result, they discovered that the antibody having the amino acid sequence provided in the present invention was very excellent in such activities, and has completed the present invention.

Therefore, the object of the present invention is to provide an antibody or antigen-binding fragment thereof that specifically binds to acid sphingomyelinase (ASM), comprising a heavy chain variable region comprising heavy chain complementarity determining region (CDR) 1 comprising the amino acid sequence defined by SEQ ID NO: 1, heavy chain CDR2 comprising the amino acid sequence defined by SEQ ID NO: 2, and heavy chain CDR3 comprising the amino acid sequence defined by SEQ ID NO: 3; and a light chain variable region comprising light chain CDR1 comprising the amino acid sequence defined by SEQ ID NO: 4, light chain CDR2 comprising the amino acid sequence defined by SEQ ID NO: 5, and light chain CDR3 comprising the amino acid sequence defined by SEQ ID NO: 6.

Another object of the present invention is to provide a polynucleotide encoding the antibody or antigen-binding fragment thereof, a vector comprising the polynucleotide, and a host cell transformed with the vector.

Another object of the present invention is to provide a pharmaceutical composition for preventing or treating neurodegenerative diseases or depression, comprising the antibody or antigen-binding fragment thereof as an active ingredient.

In addition, another object of the present invention is to provide a pharmaceutical composition for preventing or treating neurodegenerative diseases or depression, consisting of the antibody or antigen-binding fragment thereof.

In addition, another object of the present invention is to provide a pharmaceutical composition for preventing or treating neurodegenerative diseases or depression, essentially consisting of the antibody or antigen-binding fragment thereof as an active ingredient.

Another object of the present invention is to provide a composition for diagnosing neurodegenerative diseases or depression, comprising the antibody or antigen-binding fragment thereof.

In addition, another object of the present invention is to provide a composition for diagnosing neurodegenerative diseases, consisting of the antibody or antigen-binding fragment thereof.

In addition, another object of the present invention is to provide a composition for diagnosing neurodegenerative diseases, essentially consisting of the antibody or antigen-binding fragment thereof.

Another object of the present invention is to provide use of the antibody or antigen-binding fragment thereof for preparing a composition for treating neurodegenerative diseases.

Another object of the present invention is to provide a method for treating neurodegenerative diseases, comprising administering to a subject in need thereof an effective amount of a composition comprising the antibody or antigen-binding fragment thereof as an active ingredient.

Another object of the present invention is to provide use of the antibody or antigen-binding fragment thereof for preparing a composition for treating depression.

Another object of the present invention is to provide a method for treating depression, comprising administering to a subject in need thereof an effective amount of a composition comprising the antibody or antigen-binding fragment thereof as an active ingredient.

Another object of the present invention is to provide a method for diagnosing neurodegenerative diseases, comprising (a) obtaining a sample from a subject to bind thereto an antibody that specifically binds to acid sphingomyelinase (ASM); and (b) diagnosing a neurodegenerative disease based on the measured activities of ASM.

### SOLUTION TO PROBLEM

In order to achieve the above-described object of the present invention, the present invention provides an antibody or antigen-binding fragment thereof that specifically binds to acid sphingomyelinase (ASM), comprising a heavy chain variable region comprising heavy chain complementarity determining region (CDR) 1 comprising the amino acid sequence defined by SEQ ID NO: 1, heavy chain CDR2 comprising the amino acid sequence defined by SEQ ID NO: 2, and heavy chain CDR3 comprising the amino acid sequence defined by SEQ ID NO: 3; and a light chain variable region comprising light chain CDR1 comprising the amino acid sequence defined by SEQ ID NO: 4, light chain CDR2 comprising the amino acid sequence defined by SEQ ID NO: 5, and light chain CDR3 comprising the amino acid sequence defined by SEQ ID NO: 6.

In order to achieve another object of the present invention, the present invention provides a polynucleotide encoding the antibody or antigen-binding fragment thereof, a vector comprising the polynucleotide, and a host cell transformed with the vector.

In order to achieve another object of the present invention, the present invention provides a pharmaceutical composition for preventing or treating neurodegenerative diseases or depression, comprising the antibody or antigen-binding fragment thereof as an active ingredient.

In addition, the present invention provides a pharmaceutical composition for preventing or treating neurodegenerative diseases or depression, consisting of the antibody or antigen-binding fragment thereof as an active ingredient.

In addition, the present invention provides a pharmaceutical composition for preventing or treating neurodegenerative diseases or depression, essentially consisting of the antibody or antigen-binding fragment thereof as an active ingredient.

In order to achieve another object of the present invention, the present invention provides a composition for diagnosing neurodegenerative diseases, comprising the antibody or antigen-binding fragment thereof.

In addition, the present invention provides a composition for diagnosing neurodegenerative diseases, consisting of the antibody or antigen-binding fragment thereof.

In addition, the present invention provides a composition for diagnosing neurodegenerative diseases, essentially consisting of the antibody or antigen-binding fragment thereof.

In order to achieve another object of the present invention, the present invention provides use of the antibody or antigen-binding fragment thereof for preparing a composition for treating neurodegenerative diseases.

In order to achieve another object of the present invention, the present invention provides a method for treating neurodegenerative diseases, comprising administering to a subject in need thereof an effective amount of a composition comprising the antibody or antigen-binding fragment thereof as an active ingredient.

In order to achieve another object of the present invention, the present invention provides use of the antibody or antigen-binding fragment thereof for preparing a composition for treating depression.

In order to achieve another object of the present invention, the present invention provides a method for treating depression, comprising administering to a subject in need thereof an effective amount of a composition comprising the antibody or antigen-binding fragment thereof as an active ingredient.

In order to achieve another object of the present invention, the present invention provides a method for diagnosing neurodegenerative diseases, comprising (a) obtaining a sample from a subject to bind thereto an antibody that specifically binds to acid sphingomyelinase (ASM); and (b) diagnosing a neurodegenerative disease based on the measured activities of ASM.

Hereinafter, the present invention will be described in detail.

The present invention provides an antibody or antigen-binding fragment thereof that specifically binds to acid sphingomyelinase (ASM), comprising a heavy chain variable region comprising heavy chain complementarity determining region (CDR) 1 comprising the amino acid sequence defined by SEQ ID NO: 1, heavy chain CDR2 comprising the amino acid sequence defined by SEQ ID NO: 2, and heavy chain CDR3 comprising the amino acid sequence defined by SEQ ID NO: 3; and a light chain variable region comprising light chain CDR1 comprising the amino acid sequence defined by SEQ ID NO: 4, light chain CDR2 comprising the amino acid sequence defined by SEQ ID NO: 5, and light chain CDR3 comprising the amino acid sequence defined by SEQ ID NO: 6.

According to standard abbreviation conventions in the field of biochemistry, the single letter (triple letters) of amino acids used herein refers to the following amino acids: A(Ala): alanine; C(Cys): cysteine; D(Asp): aspartic acid; E(Glu): glutamic acid; F(Phe): phenylalanine; G(Gly): glycine; H(His): histidine; I(Ile): isoleucine; K(Lys): lysine; L(Leu): leucine; M(Met): methionine; N(Asn): asparagine; O(Ply): pyrrolysine; P(Pro): proline; Q(Gln): glutamine; R(Arg): arginine; S(Ser): serine; T(Thr): threonine; U(Sec): selenocysteine, V(Val): valine; W(Trp): tryptophan; and Y(Tyr): tyrosine.

As used herein, the term "expression" refers to the production of a protein or nucleic acid in a cell.

As used herein, the term "host cell" refers to a prokaryotic or eukaryotic cell comprising heterologous DNA introduced into cells by any means (e.g., electroshock, calcium phosphatase precipitation, microinjection, transformation, and viral infection).

As used herein, the term "protein" refers to a polymer of amino acid residues which is used interchangeably with "polypeptide" and, e.g., commonly found in a protein in its natural state.

As used herein, the term "nucleic acid," "DNA sequence," or "polynucleotide" refers to deoxyribonucleotide or ribonucleotide in a single- or double-stranded form. Unless otherwise limited, the terms also comprise known analogs of natural nucleotide that hybridizes to a nucleic acid in a manner similar to a naturally occurring nucleotide.

The heavy chain and light chain of an antibody is structurally divided into variable regions and constant regions depending on the variability of the amino acid sequence. The constant region of the heavy chain consists of 3 or 4 heavy chain constant regions (e.g., CH1, CH2 and CH3 (IgA, IgD and IgG antibodies), and CH4 (IgE and IgM antibodies)) depending on the type of the antibody, and the constant region of the light chain consists of a single light chain constant region, CL. The variable regions of the heavy chain and light chain each consist of one domain, the heavy chain variable region (VH) or the light chain variable region (VL). The light chain and heavy chain have their respective variable region and constant region aligned side by side and connected by a single shared disulfide bond, and the heavy chains of the two molecules bound to the light chain are connected by two shared disulfide bonds, forming a whole antibody. The whole antibody specifically binds to an antigen through the variable regions of the heavy chain and light chain. Since the whole antibody comprises two pairs of heavy chain and light chain (HC/LC), the whole antibody of a single molecule has bivalent monospecificity that binds to the same two antigens through two variable regions.

The variable region comprising the region that the antibody binds to the antigen is subdivided into a framework region (FR) with a low sequence variability and a complementary determining region (CDR) which is a hypervariable region with a high sequence variability. The VH and VL each have three CDRs and four FRs arranged from the N-terminus to the C-terminus in the order of FR1-CDR1-FR2-CDR2-FR3-CDR3-FR4. The CDR with the highest sequence variability in the variable region of the antibody is the region that directly binds to the antigen and is most important for the antigen specificity of the antibody.

The monoclonal antibody of the present invention represents an antibody obtained from a population of substantially homogeneous antibodies. That is, the individual antibodies constituting the population are identical except for possible naturally occurring mutations, which may be present in small quantities. The monoclonal antibody binds very specifically to a single antigenic epitope.

As used herein, the term "monoclonal" refers to the characteristic of an antibody that the antibody is obtained from a group of substantially homologous antibodies, and does not necessarily mean that the antibody must be produced by a specific method. For example, the monoclonal antibody of the present invention may be produced by the hybridoma method first disclosed in a reference (see Kohler et al., Nature (1975), 256: 495), or by the recombinant DNA method (see U.S. Patent No. 4,816,567). In addition, for example, it may be isolated from a phage antibody library using the techniques described in references (see Clackson et al., Nature (1991), 352: 624-628; Marks et al., J. Mol. Biol. (1991), 222: 581-597; and Presta, J. Allergy Clin. Immunol. (2005), 116: 731).

The antibody of the present invention specifically comprises a chimeric antibody. In such case, although a portion of the heavy chain and/or light chain originates from a particular species, or has a sequence identical or homologous to the corresponding sequence of a particular antibody, the remaining portion may originate from another species, or have a sequence identical or homologous to the corresponding sequence of another antibody, as long as the antibody of the present invention exhibits desired biological activities (e.g., selective binding to an ASM protein) (U.S. Patent No. 4,816,567).

A humanized antibody is an antibody that comprises the sequences of both human and non-human (e.g., murine and rat) antibodies. In general, the remaining regions except the epitope-binding regions (CDRs) are those of a human antibody, and the epitope-binding regions (CDRs) may comprise the sequences of non-human origin. A fully human antibody refers to an antibody comprising only human immunoglobulin protein sequences, and may be produced in mice, mouse cells, or hybridomas derived from mouse cells, or may be produced by phage display methods.

The hybridoma cells may be produced using methods commonly known in the art. Specifically, the hybridoma cells may be prepared by the process of immunizing animals with an ASM protein which is an immunogen, fusing B cells, which are antibody producing cells derived from the immunized animals, with myeloma cells to prepare hybridomas, and selecting therefrom a hybridoma that produces a monoclonal antibody that specifically binds to the peptide. The immunized animals may comprise, in addition to mice, animals such as goats, sheep, guinea pigs, rats, and rabbits.

A method for immunizing the immunized animals may comprise a method commonly known in the art. For example, immunizing mice may be performed by the method of emulsifying 1 to 100 µg of an immunogen at a time with the same amount of physiological saline and/or an antigen adjuvant such as Freund's adjuvant, and inoculating same subcutaneously or intraperitoneally in the abdomen of the immunized animals 2 to 6 times every 2 to 5 weeks. 3 to 5 days after the final immunization of the immunized animals, the spleen or lymph nodes may be removed, and the B cells contained in their tissues may be fused with myeloma cells in the presence of a fusion promoter according to a cell fusion method commonly known in the art. The fusion promoter may comprise, for example, a substance such as polyethylene glycol (PEG). The myeloma cells may comprise, for example, mouse-derived cells such as P3U1, NS-1, P3x63Ag8.653, and Sp2/0-Ag14, and rat-derived cells such as AG1 and AG2. In addition, the cell fusion method commonly known in the art may be performed, for example, by mixing B cells and myeloma cells at a ratio of 1:1 to 10:1, and adding thereto PEG with a molecular weight of 1,000 to 6,000 at a concentration of 10 to 80%, followed by incubation at 30 to 37°C for 1 to 10 minutes. In addition, hybridomas that produce monoclonal antibodies that specifically bind to the immunogenic peptide may be selected, for example, by culturing hybridomas in a selective medium such as HAT medium in which only hybridomas are viable, and measuring the activities of the antibodies in the hybridoma culture supernatant by using a method such as ELISA. Finally, hybridomas that produce monoclonal antibodies that specifically bind to the immunogenic peptide may be selected by repeating cloning for the hybridomas by methods such as limiting dilution.

In addition, the monoclonal antibodies or antigen-binding fragments thereof provided by the present invention can generate human antibodies and antibody fragments in vitro from immunoglobulin variable region gene repertoires from non-immunized donors, by using phage display technology. According to this technology, antibody variable region genes are in-frame cloned into the major or minor envelope protein of filamentous bacteriophages, such as M13 and fd, and functional antibody fragments are displayed on the surface of the phage particles. Because filamentous particles contain a single-stranded DNA copy of the phage genome, selection based on the functional properties of the antibody results in the selection of genes encoding antibodies that exhibit such properties. Therefore, the phage mimics some characteristics of B-cells. Phage display may be performed in a variety of formats. Several sources of variable region-gene segments may be used for the phage display. The reference [Clackson et al., Nature (1991), 352:624-628] discloses that a diverse array of anti-oxazolone antibodies was isolated from a small random combinatorial library of variable region genes derived from the spleens of immunized mice. A repertoire of variable region genes from non-immunized human donors may be constructed, and antibodies to a diverse array of antigens (comprising autoantigens) may be isolated by essentially using techniques commonly known in the art [see U.S. Patent Nos. 5,565,332 and 5,573,905].

In the present invention, the feature that the light chain or heavy chain variable region comprises a particular amino acid sequence indicates all of the cases that it comprises the entire amino acid sequence, has the entire amino acid sequence, or consists of the amino acid sequence.

In one aspect of the present invention, the antibody or antigen-binding fragment thereof may comprise a heavy chain variable region (VH) comprising the amino acid sequence defined by SEQ ID NO: 13, and a light chain variable region (VL) comprising the amino acid sequence defined by SEQ ID NO: 15.

The type of the antibody according to the present invention is not specifically limited as long as it specifically recognizes the ASM proteins. As a specific example, the antibody may be selected from the group consisting of IgG, IgA, IgM, IgE, and IgD.

In the present invention, the antigen-binding fragment of the antibody refers to the fragment of the antibody that maintains binding ability to the ASM proteins, and the fragment preferably retains at least 20%, 50%, 70%, 80%, 90%, 95% or 100% or more of the protein affinity of the parent antibody. Specifically, it may be in the form of diabody, Fab, Fab', F(ab)2, F(ab')2, Fv and scFv.

The fragment antigen-binding (Fab) is an antigen-binding fragment of an antibody and comprises one variable domain and one constant domain of each of the heavy and light chain. The F(ab')2 is a fragment produced by hydrolyzing an antibody with pepsin, and comprises two Fabs connected by a disulfide bond at the heavy chain hinge. The F(ab') is a monomeric antibody fragment in which a heavy chain hinge is added to Fab isolated by reducing the disulfide bond of the F(ab')2 fragment. The variable fragment (Fv) is an antibody fragment consisting only of the variable regions of each heavy chain and light chain. The single chain variable fragment (scFv) is a recombinant antibody fragment in which the heavy chain variable region (VH) and light chain variable region (VL) are connected by a flexible peptide linker. The diabody refers to a fragment in which the VH and VL of the scFv are connected by a very short linker and cannot bind to each other, and bind to the VL and VH, respectively, of another scFv of the same type to form a dimer.

The antibody or fragment thereof of the present invention may comprise conservative amino acid substitutions that do not substantially alter its biological activities (referred to as conservative variants of the antibody). Regarding such amino acid substitutions, the above description may be referred to.

In addition, the antibody or fragment thereof of the present invention described above may be conjugated to an enzyme, a fluorescent substance, a radioactive substance, a protein, and the like, but the present invention is not limited thereto. In addition, methods for conjugating such substances to an antibody are commonly known in the art.

The present invention also provides a polynucleotide encoding the antibody or antigen-binding fragment thereof.

In the present invention, the term "polynucleotide" may be described as an oligonucleotide or nucleic acid and comprises DNA molecules (e.g., cDNA and genomic DNA), RNA molecules (e.g., mRNA), analogues of the DNA or RNA produced by using nucleotide analogs (e.g., peptide nucleic acids and non-naturally occurring nucleotide analogs), and hybrids thereof. The polynucleotide may be single-stranded or double-stranded. The polynucleotide refers to a base sequence encoding an antibody comprising a heavy chain and a light chain having CDRs specific to the residues of the ASM protein, or VH and VL.

The polynucleotide encoding the antibody or fragment thereof of the present invention may be obtained by using oligonucleotide synthesis techniques commonly known in the art, such as polymerase chain reaction (PCR), based on DNA sequences encoding part or all of the heavy chain and light chain of the antibody, or on the corresponding amino acid sequences.

In one embodiment of the present invention, the polynucleotide encoding the antibody or antigen-binding fragment thereof may comprise the nucleotide sequences shown in Table 1 and/or Table 2 below.

The present invention also provides a vector comprising the polynucleotide.

The "vector" of the present invention is used for the purpose of replication or expression of the polynucleotide of the present invention for recombinant production of the antibody or fragment thereof of the present invention, and generally comprises at least one of a signal sequence, origin of replication, one or more marker genes, an enhancer element, a promoter, and a transcription termination sequence. The vector of the present invention may preferably be an expression vector, and more preferably may be a vector comprising the polynucleotide of the present invention operably linked to a control sequence, for example, a promoter.

A plasmid which is a type of vector refers to a linear or circular double-helix DNA molecule to which external polynucleotide fragments may be combined. Another type of vector is a viral vector (e.g., replication defective retroviruses, adenoviruses, and adeno-associated viruses), in which additional DNA fragments may be introduced into the viral genome. Certain vectors are capable of autonomous replication within the host cell into which they are introduced (e.g., bacterial vectors, comprising bacterial origin and episomal mammalian vectors). Other vectors (e.g., non-episomal mammalian vectors) are integrated into the genome of the host cell by introduction into the host cell and, therefore, replicated along with the host genome.

In the present invention, the "vector" may be understood to have the same meaning as an "expression vector," which is a type of vector capable of expressing the polynucleotide. One polynucleotide sequence is "operably linked" to a regulatory sequence in the case that the regulatory sequence affects the expression (e.g., level, timing or location of expression) of the polynucleotide sequence. The regulatory sequence is a sequence that affects the expression (e.g., level, timing or location of expression) of the nucleic acid to which it is operably linked. The regulatory sequence may, for example, exert its influence directly on the regulated nucleic acid or through the action of one or more other molecules (e.g., polypeptides that bind to the regulatory sequence and/or the nucleic acid). The regulatory sequences comprise promoters, enhancers and other expression-regulating elements.

The present invention also provides cells transformed with the vector.

The type of cells of the present invention is not particularly limited as long as they can be used to express the polynucleotide encoding the antibody or fragment thereof contained in the expression vector of the present invention. The cells (host cells) transformed with the expression vector according to the present invention may be prokaryotes (e.g., Escherichia coli); eukaryotes (e.g., yeast and other fungi); plant cells (e.g., tobacco and tomato plant cells), animal cells (e.g., human cells, monkey cells, hamster cells, rat cells, and mouse cells), insect cells, or hybridomas derived therefrom. Preferably, they may be cells derived from mammals such as human beings.

Prokaryotes suitable for the object of the present invention comprise Gram-negative or Gram-positive organisms, for example, Enterobacteriaceae (e.g., Escherichia (such as E.coli), Enterobacter, Erwinia, Klebsiella, Proteus, Salmonella (such as Salmonella typhimurium), Serratia (such as Serratia marcescans and Shigella), and Bacilli (such as B. subtilis and B. licheniformis)), Pseudomonas (e.g., P. aeruginosa), and Streptomyces. The cells of the present invention are not particularly limited as long as they can expressing the vector of the present invention, but may preferably be E. coli.

The eukaryotes as cells of the present invention may comprise Saccharomyces cerevisiae as the most commonly used. However, they may comprise many other genera, species and strains, for example, Schizosaccharomyces pombe; Kluyveromyces host (e.g., K. lactis, K. fragilis (ATCC 12,424), K. bulgaricus (ATCC 16,045), K. wickeramii (ATCC 24,178), K. waltii (ATCC 56,500), K.drosophilarum (ATCC 36,906), K. thermotolerans, and K. marxianus); yarrowia (EP 402,226); Pichia pastoris (EP 183,070); Candida; Trichoderma reesia (EP 244,234); Neurospora crassa; Schwanniomyces (e.g., Schwanniomyces occidentalis); and Filamentous fungi (e.g., Neurospora, Penicillium, Tolypocladium, and Aspergillus hosts (e.g., A. nidulans and A. niger)), but the present invention is not limited thereto.

The term "transformation" refers to the modification of the genotype of a host cell by the introduction of an exogenous polynucleotide, and refers to the introduction of the exogenous polynucleotide into the host cell regardless of the method used for the transformation. An exogenous polynucleotide introduced into a host cell may remain integrated into the genome of the host cell or may remain unintegrated, and the present invention comprises both.

A recombinant expression vector capable of expressing the antibody or antigen-binding fragment thereof according to the present invention may be transformed by being introduced into a cell for producing an antibody or a fragment thereof by methods commonly known in the art, for example, transient transfection, microinjection, transduction, cell fusion, calcium phosphate precipitation, liposomemediated transfection, DEAE dextran-mediated transfection, polybrene-mediated transfection, electroporation, gene gun, and known methods for introducing nucleic acids into cells, but the present invention is not limited thereto.

In addition, the cells of the present invention are cultured cells that may be transformed or transfected with the polynucleotide or vector comprising same of the present invention, which may be continuously expressed in the host cell. The recombinant cells refer to cells that have been transformed or transfected with a polynucleotide to be expressed. The cells of the present invention may also be cells that comprise the polynucleotide of the present invention but do not express same at a desired level, unless regulatory sequences are introduced into the cells so as to be operably linked to the polynucleotide.

The cells of the present invention may be cultured in various media. Commercially available media, for example, Ham's F1O (Sigma-Aldrich Co., St. Louis, MO), minimal essential medium (MEM, Sigma-Aldrich Co.), RPMI-1640 (Sigma-Aldrich Co.), and Dulbecco's modified Eagle's medium (DMEM, Sigma-Aldrich Co.), are suitable for culturing cells. Hormones and/or other growth factors, salts, buffers, nucleotides, antibiotics, trace elements and glucose or equivalent energy sources may be added to the media, if necessary.

The present invention provides a method for producing an antibody that binds to WRS, or a fragment thereof, comprising culturing the cells under conditions in which polynucleotides are expressed to produce polypeptides comprising light chain and heavy chain variable regions, and recovering the polypeptide from the cells or the culture medium in which they were cultured.

The cells used in the production method of the present invention are as described above, and comprise the polynucleotide encoding the antibody of the present invention. The polypeptide of the production method may be the antibody or fragment thereof of the present invention per se, and may have an additional amino acid sequence other than that of the antibody or fragment thereof of the present invention.

In such case, it may be removed from the antibody or fragment thereof of the present invention by using methods commonly known to a person skilled in the art. The composition of a culture medium and culturing conditions may vary depending on the type of cells, which may be appropriately selected and adjusted by a person skilled in the art.

The antibody molecule may accumulate in the cytoplasm of a cell, be secreted from a cell, or be targeted to the periplasm or extracellular medium (supernatant) by an appropriate signal sequence, and is preferably targeted to the periplasm or extracellular medium. In addition, it is preferred that the produced antibody molecule is refolded by using a method commonly known to a person skilled in the art and has a functional conformation. The recovery of the polypeptide may change depending on the characteristics of the produced polypeptide and the characteristics of the cell, which may be appropriately selected and adjusted by a person skilled in the art.

The polypeptide may be produced intracellularly, in the periplasmic space, or secreted directly into the medium. If the polypeptide is produced within a cell, the cell may be destroyed to release a protein as a first step. Particulate debris, host cells or dissolved fragments are removed, for example, by centrifugation or ultrafiltration. When the antibody is secreted into the medium, supernatants from such expression systems are typically first concentrated, by using commercially available protein concentration filters (e.g., Amicon and Millipore Pellicon ultrafiltration units). Protease inhibitors such as PMSF may be contained in any preceding step to inhibit proteolysis, and antibiotics may be contained to prevent the growth of accidental contaminants. The antibody prepared from the cells may be purified, for example, by using hydroxyapatite chromatography, gel electrophoresis, dialysis, and affinity chromatography, and the antibody of the present invention may preferably be purified through affinity chromatography.

The antibody or antigen-binding fragment thereof of the present invention is very effective in inhibiting the activities of ASM, and can be used as a preventive or therapeutic agent for various neurodegenerative diseases such as Alzheimer's disease which may be caused by ASM.

Accordingly, the present invention provides a pharmaceutical composition for preventing or treating neurodegenerative diseases and depression, comprising the antibody or antigen-binding fragment thereof as an active ingredient.

In the present invention, it may be characterized in that the neurodegenerative disease is at least one type selected from the group consisting of Alzheimer's disease, Parkinson's disease, progressive supranuclear palsy, multiple system atrophy, olivopontocerebellar atrophy (OPCA), Shay-Drager syndrome, striato-substantia nigra degeneration, Huntington's disease, amyotrophic lateral sclerosis (ALS), essential tremor, cortico-basal degeneration, diffuse Lewy body disease, Parkinson-ALS-dementia complex, Niemann-Pick disease, Pick's disease, cerebral ischemia, and cerebral infarction, but the present invention is not limited thereto.

The pharmaceutical composition according to the present invention may comprise only the antibody or antigen-binding fragment thereof, or may be formulated in a suitable form, together with a pharmaceutically acceptable carrier, and may further comprise an excipient or diluent. The carrier comprises all kinds of solvents, dispersion media, oil-in-water or water-in-oil emulsions, aqueous compositions, liposomes, microbeads and microsomes.

The pharmaceutically acceptable carrier may further comprise, for example, carriers for oral administration or carriers for parenteral administration. The carriers for oral administration may comprise lactose, starch, cellulose derivatives, magnesium stearate and stearic acid. In addition, they may comprise various drug delivery substances used for oral administration. In addition, the carriers for parenteral administration may comprise water, suitable oils, saline solutions, aqueous glucose and glycols, and may further comprise stabilizers and preservatives. Suitable stabilizers are antioxidants such as sodium bisulfite, sodium sulfite and ascorbic acid. Suitable preservatives are benzalkonium chloride, methyl- or propyl-paraben and chlorobutanol. The pharmaceutical composition of the present invention may comprise, in addition to the ingredients, lubricants, wetting agents, sweeteners, flavoring agents, emulsifiers, and suspending agents. For other pharmaceutically acceptable carriers and preparations, reference may be made to those known in the art.

The composition of the present invention may be administered to mammals comprising human beings by any method. For example, it may be administered orally or parenterally. The parenteral administration method may be intravenous, intramuscular, intraarterial, intramedullary, intrathecal, intracardiac, transdermal, subcutaneous, intraperitoneal, intranasal, enteral, topical, sublingual or rectal administration, but the present invention is not limited thereto.

The pharmaceutical composition of the present invention may be formulated as a formulation for oral administration or parenteral administration according to the administration routes described above.

For the formulation for oral administration, the composition of the present invention may be formulated into powders, granules, tablets, pills, sugar-coated tablets, capsules, solutions, gels, syrups, slurries, and suspensions, by using a method commonly known in the art. For example, the formulation for oral administration may be formulated by mixing the active ingredient with solid excipients, grinding the mixture, adding thereto suitable auxiliaries, and processing same into a granule mixture to obtain tablets or sugar tablets. Examples of suitable excipients may comprise sugars (such as lactose, dextrose, sucrose, sorbitol, mannitol, xylitol, erythritol, and maltitol), starches (such as corn starches, wheat starches, rice starches, and potato starches), celluloses (such as celluloses, methyl celluloses, sodium carboxymethylcelluloses, and hydroxypropylmethyl-celluloses), and fillers (such as gelatin and polyvinylpyrrolidone). In addition, in some cases, cross-linked polyvinylpyrrolidone, agar, alginic acid, or sodium alginate may be added as a disintegrant. Furthermore, the pharmaceutical composition of the present invention may further comprise anticoagulants, lubricants, wetting agents, flavorings, emulsifiers, and preservatives.

For the formulation for parenteral administration, the composition of the present invention may be formulated into the forms of injections, creams, lotions, external ointments, oils, moisturizers, gels, and aerosols. and nasal inhalants, by methods known in the art. These formulations are described in formulas generally known in all pharmaceutical chemistry.

The total effective amount of the composition of the present invention may be administered to a patient in a single dose, or may be administered by a fractionated treatment protocol in which multiple doses are administered over a long period of time. The pharmaceutical composition of the present invention comprises an active ingredient whose amount may vary depending on the severity of a disease. The total dose of the pharmaceutical composition of the present invention may be preferably about 0.01 µg to 10,000 mg per kg of patient body weight per day, and most preferably 0.1 µg to 500 mg per kg of patient body weight per day. However, the effective dose the pharmaceutical composition for a patient is determined in consideration of various factors, e.g., patient's age, weight, health status, gender, disease severity, diet and excretion rate, as well as a formulation method, an administration route and the number of treatments. Considering them, a person skilled in the art may determine an appropriate effective dose of the composition of the present invention. The pharmaceutical composition according to the present invention is not particularly limited in formulations, administration routes, and administration methods thereof as long as the composition exhibits the effects of the present invention.

The antibody or antigen-binding fragment thereof of the present invention specifically binds to an ASM protein and, therefore, is useful, e.g., for diagnosis or analysis for detecting and quantifying the expression of ASM protein in specific cells, tissues, or serum.

For such purpose, the antibody or antigen-binding fragment thereof may generally be labeled with a detectable moiety.

For example, it may be labeled with a radioactive isotope or fluorescent label by using techniques commonly known in the art. Radioactivity may be measured, for example, by scintillation counting, and fluorescence may be quantified by using a fluorometer. Alternatively, a variety of enzyme-substrate labels are available, and examples of the enzymatic labels comprise luciferase (e.g., drosophila luciferase and bacterial luciferase (U.S. Patent No. 4,737,456)), Peroxidase (e.g., luciferin, 2,3-dihydrophthalazindiones, malate dehydrogenase, urase, horseradish peroxidase (HRPO)), alkaline phosphatase, β-galactosidase, glucoamylase, lysozyme, saccharide oxidase (e.g., glucose oxidase, galactose oxidase, and glucose-6-phosphate dehydrogenase), heterocyclic oxidase (e.g., uricase and xanthine oxidase), lactoperoxidase, microperoxidase, and the like. Techniques for conjugating enzymes to antibodies are known in the art.

A label may be indirectly conjugated to an antibody by using a variety of known techniques. For example, an antibody may be conjugated to biotin, and any of the three broad categories mentioned above may be conjugated to avidin and vice versa. Biotin binds selectively to avidin, so this label may be conjugated to an antibody in such indirect manner. Alternatively, in order to achieve indirect conjugation of the label to an antibody, the antibody may be conjugated to a small hapten (e.g., digoxin), and one of the different types of labels mentioned above may be conjugated to an antihapten antibody (e.g., an anti-dioxin antibody). Therefore, indirect conjugation of the label to the antibody may be achieved.

The antibody or antigen-binding fragment thereof of the present invention may be used in any known analysis method such as competitive binding assays, direct and indirect sandwich assays, and immunoprecipitation assays.

The antibody or antigen-binding fragment thereof of the invention may be used in a diagnostic kit, i.e., a diagnostic kit for performing diagnostic analyses, which is a combination of reagents packaged in pre-specified amounts together with instructions for use. If the antibody is labeled with an enzyme, the kit may comprise a substrate and a cofactor required by the enzyme as a substrate precursor for providing a chromophore or fluorophore. In addition, other additives such as stabilizers and buffers (e.g., blocking buffers and lysis buffers) may be contained therein. The relative amounts of the various reagents may be varied widely in order to provide a concentration in solution of a reagent that sufficiently optimizes the sensitivity of the assays. The reagent may be provided as a dry powder, usually lyophilized, comprising excipients that might provide a reagent solution having an appropriate concentration upon dissolution.

Meanwhile, as described above, it has been reported that the expression level of ASM protein is increased in biological samples obtained from patients with neurodegenerative diseases, as compared to normal people. Therefore, analyzing the expression level of ASM protein may diagnose neurodegenerative diseases and evaluate the disease progression status and prognosis before and after treatment.

Accordingly, the present invention provides a composition for diagnosing neurodegenerative diseases, comprising the antibody or antigen-binding fragment thereof.

In addition, the present invention provides a kit for diagnosing neurodegenerative diseases, comprising the antibody or antigen-binding fragment thereof.

The kit for diagnosis of the present invention may comprise, in addition to the antibody or antigen-binding fragment thereof, a composition having one or more different components suitable for an analysis method, a solution, or a device.

More specifically, the kit may be a diagnostic kit. Such kit may comprise an antibody specific for control protein. In addition, the kit comprises reagents capable of detecting bound antibodies, such as labeled secondary antibodies, chromophores, enzymes (in the form of being conjugated with antibodies) and their substrates, or other substances that may bind to antibodies. In addition, the kit of the present invention may comprise a washing solution or an eluent.

The present invention provides use of the antibody or antigen-binding fragment thereof for preparing a composition for treating neurodegenerative diseases.

The present invention provides a method for treating neurodegenerative diseases, comprising administering to a subject in need thereof an effective amount of a composition comprising the antibody or antigen-binding fragment thereof as an active ingredient.

The present invention provides use of the antibody or antigen-binding fragment thereof for preparing a composition for treating depression.

The present invention provides a method for treating depression, comprising administering to a subject in need thereof an effective amount of a composition comprising the antibody or antigen-binding fragment thereof as an active ingredient.

The present invention provides a method for diagnosing neurodegenerative diseases, comprising (a) obtaining a sample from a subject to bind thereto an antibody that specifically binds to acid sphingomyelinase (ASM); and (b) diagnosing a neurodegenerative disease based on the measured activities of ASM.

The term "effective amount" of the present invention refers to an amount that, when administered to a subject, produces the effect of improving, treating, detecting, diagnosing degenerative neurological diseases or depression, or suppressing or reducing the diseases, and the term "subject" may be an animal, preferably a mammal, especially an animal comprising a human being, and may be cells, tissues, organs, etc. derived from animals. The subject may be a patient in need of the effect.

The term "treatment" of the present invention comprehensively refers to alleviation of neurodegenerative diseases or depression, or symptoms caused by the diseases, which may comprise curing, substantially preventing, or alleviating the condition of the diseases, and comprises alleviating, curing or preventing one or most symptoms resulting from the diseases, but the present invention is not limited thereto.

As used herein, the term "comprising" is used to have the same meaning as "including" or "characterized by." The composition or method according to the present invention does not exclude additional components or steps of the method that are not specifically mentioned. In addition, the term "consisting of" indicates that additional elements, steps, or components that are not separately stated are excluded. The term "essentially consisting of" indicates that the scope of the composition or method may comprise, in addition to the substances or steps described herein, substances or steps that do not substantially affect the basic properties thereof.

### ADVANTAGEOUS EFFECTS OF INVENTION

The ASM antibody according to the present invention has very high sensitivity and specificity for ASM to be used to diagnose neurodegenerative diseases through detection of ASM, and is also very effective in inhibiting the activities of ASM to be used in the development of preventive or therapeutic agents for various neurodegenerative diseases such as Alzheimer's disease, and depression.

### BRIEF DESCRIPTION OF DRAWINGS

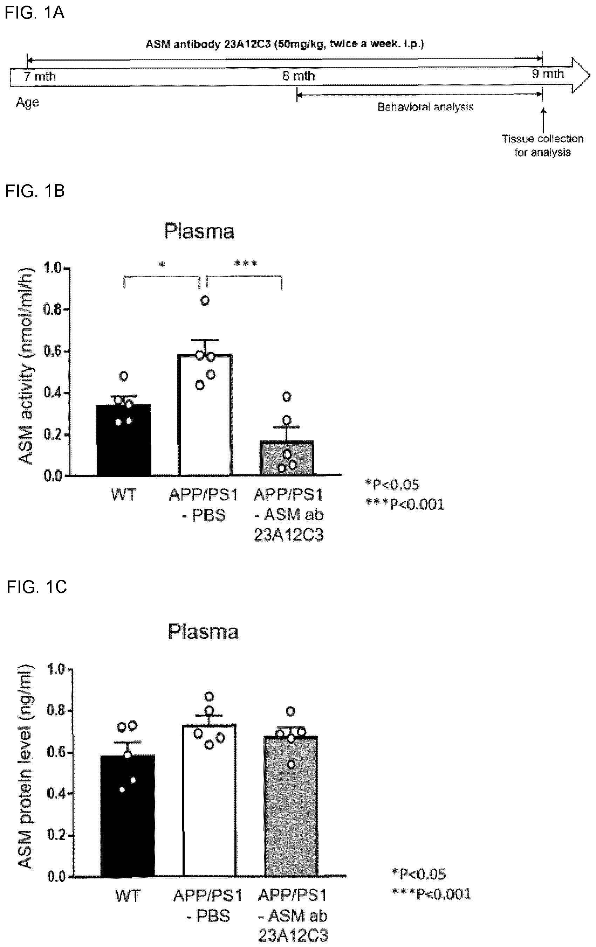

FIGS. 1A to 1C are diagrams showing the overview of experiments performed to determine the effect of inhibition of ASM activities on Alzheimer's disease by injection of an anti-ASM antibody (ASM-ab: monoclonal ASM antibody 23A12C3) (FIG. 1A), and changes in ASM activities in the serum of mice (FIG. 1B) and ASM protein concentrations (FIG. 1C) after injection of PBS or ASM-ab in Alzheimer's animal models (n=5/group) (WT: wild type, AD: Alzheimer's animal model (APP/PS1 mouse)).

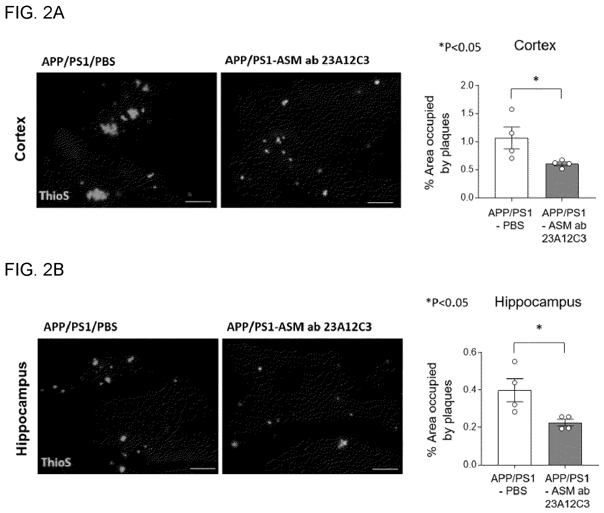

FIGS. 2A and 2B show the results of quantifying the areas occupied by immunofluorescent staining of Thioflavin S (ThioS, fibrillar amyloid beta plaque) and fibrillar amyloid beta plaque in the cortex (FIG. 2A) and hippocampus (FIG. 2B) of Alzheimer's animal models injected with PBS or ASM-ab 23A12C3 (n=4/group) (WT: wild type, AD: Alzheimer's animal model (APP/PS1 mouse)).

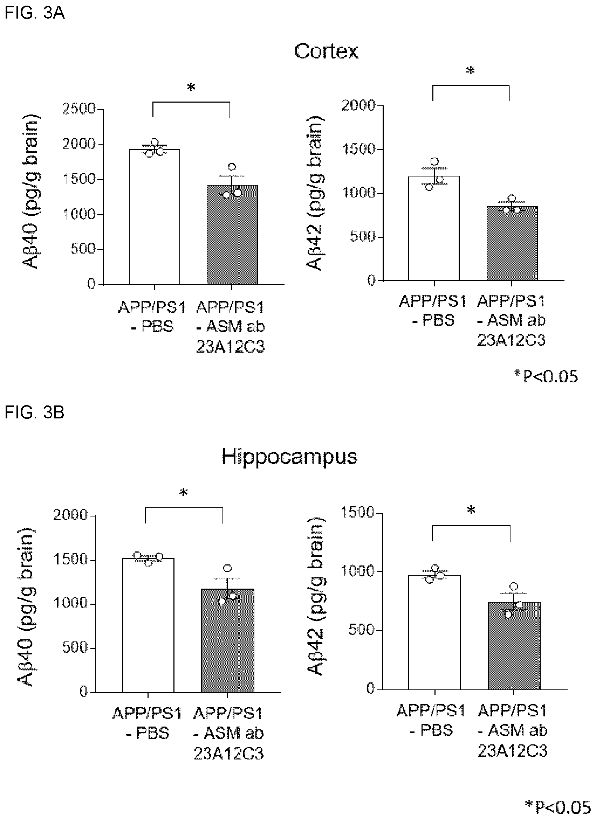

FIGS. 3A and 3B show the results of measuring the accumulation of Aβ40 or Aβ42 in the cortex (FIG. 3A) and hippocampus (FIG. 3B) of Alzheimer's animal models injected with PBS or ASM-ab 23A12C3 by ELISA (n=3/group) (WT: wild type, AD: Alzheimer's animal model (APP/PS1 mouse)).

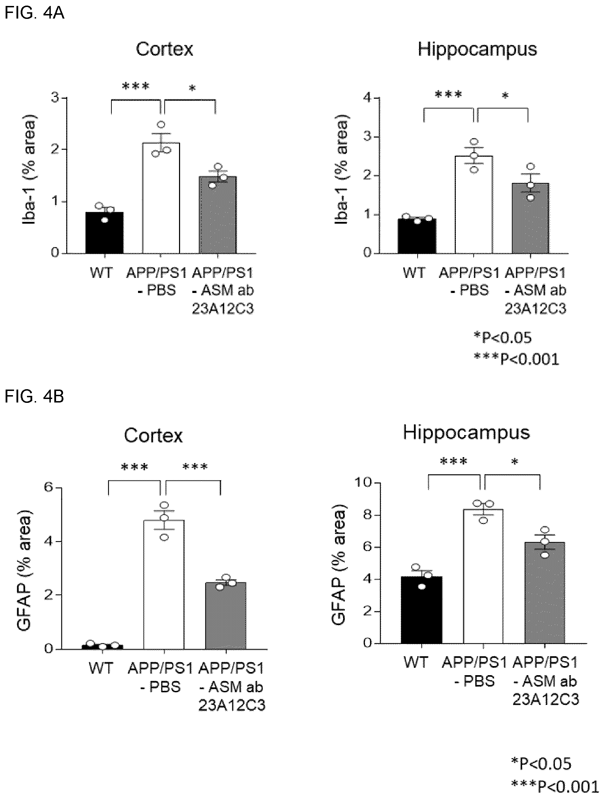

FIGS. 4A and 4B show the results of confirming that increased neuroinflammation in Alzheimer's animal models was reduced by ASM-ab 23A12C3 injection (WT: wild type, AD: Alzheimer's animal model (APP/PS1 mouse)).
FIG. 4A shows the results of quantifying the percentage of microglial cells (Iba-1) in the cortex and hippocampus of wild-type mice, and Alzheimer's animal models injected with PBS or ASM-ab 23A12C3 (n=3/group).
FIG. 4B shows the results of quantifying the percentage of astrocytes (GFAP) in the cortex and hippocampus of wild-type mice, and Alzheimer's animal models injected with PBS or ASM-ab 23A12C3 (n=3/group).
FIGS. 5A to 5D show results on the degree of recovery of learning and cognitive function in Alzheimer's animal models injected with PBS or ASM-ab 23A12C3 (WT: wild type, AD: Alzheimer's animal model (APP/PS1 mouse)).

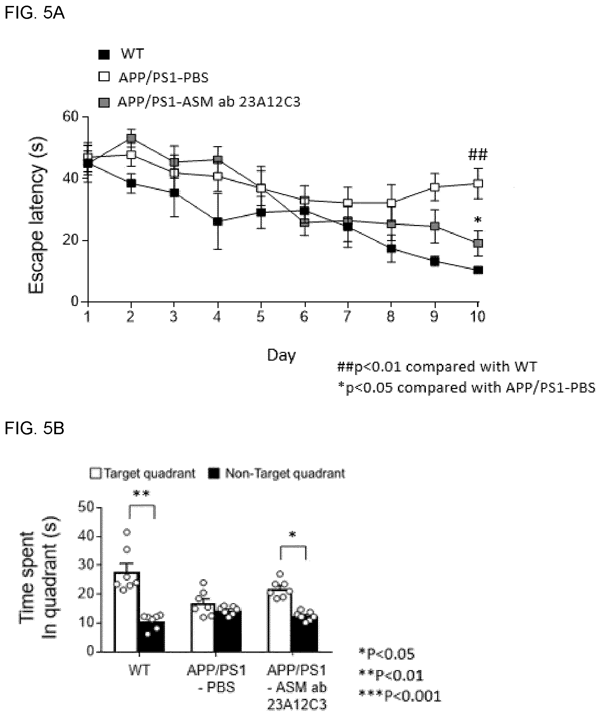

FIG. 5A shows the results of evaluating learning and memory in wild-type mice (n=7), Alzheimer's animal models injected with PBS (n=7), and Alzheimer's animal models injected with ASM-ab 23A12C3 (n=7), through the Morris Watermaze test.
FIG. 5B shows results on the time spent on the target platform on Day 11 of testing.

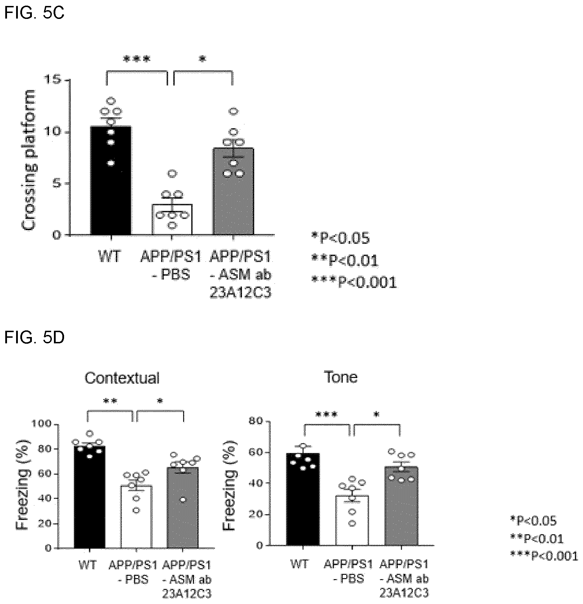

FIG. 5C shows the number of entries into the target area of the target platform on Day 11 of testing.
FIG. 5D shows the results of evaluating memory for space and sound in wild-type mice (n=7), Alzheimer's animal models injected with PBS (n=7), and Alzheimer's animal models injected with ASM-ab 23A12C3 (n=7), through a fear conditioning test.

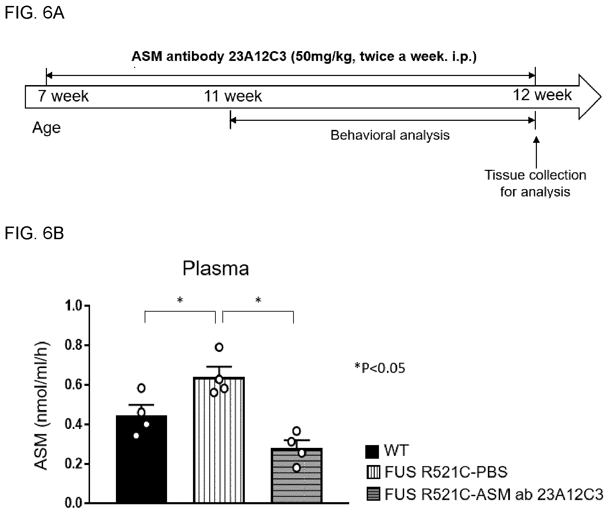

FIGS. 6A and 6B are diagrams showing the overview of experiments performed to determine the effect of inhibition of ASM activities on ALS disease by injection of an anti-ASM antibody (ASM-ab: monoclonal ASM antibody 23A12C3) (FIG. 6A), and a change in ASM activities in the serum of mice after injection of PBS or ASM-ab 23A12C3 in ALS animal models (FIG. 6B) (n=4/group) (WT: wild type, FUS R521C: ALS mouse model).

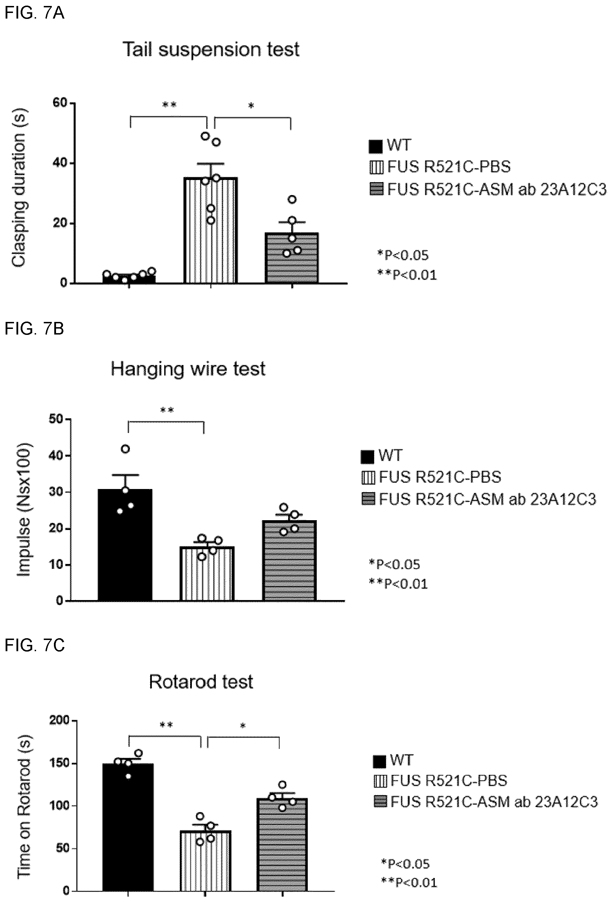

FIGS. 7A to 7C show results on the degree of recovery of motor function in ALS animal models injected with PBS or ASM-ab 23A12C3 through tail suspension test (FIG. 7A), hanging wire test (FIG. 7B), and Rotarod test (FIG. 7C) (n=4-6/group) (WT: wild type, FUS R521C: ALS mouse model).

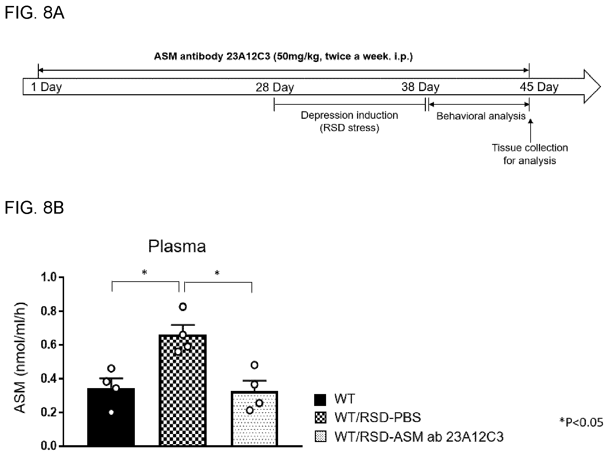

FIGS. 8A and 8B are diagrams showing the overview of experiments performed to determine the effect of inhibition of ASM activities on depression by injection of an anti-ASM antibody (ASM-ab: monoclonal ASM antibody 23A12C3) (FIG. 8A), and a change in ASM activities in the serum of mice after injection of PBS or ASM-ab 23A12C3 in depression animal models (FIG. 8B) (n=4/group) (WT: wild type, WT/RSD: depression-induced mouse model).

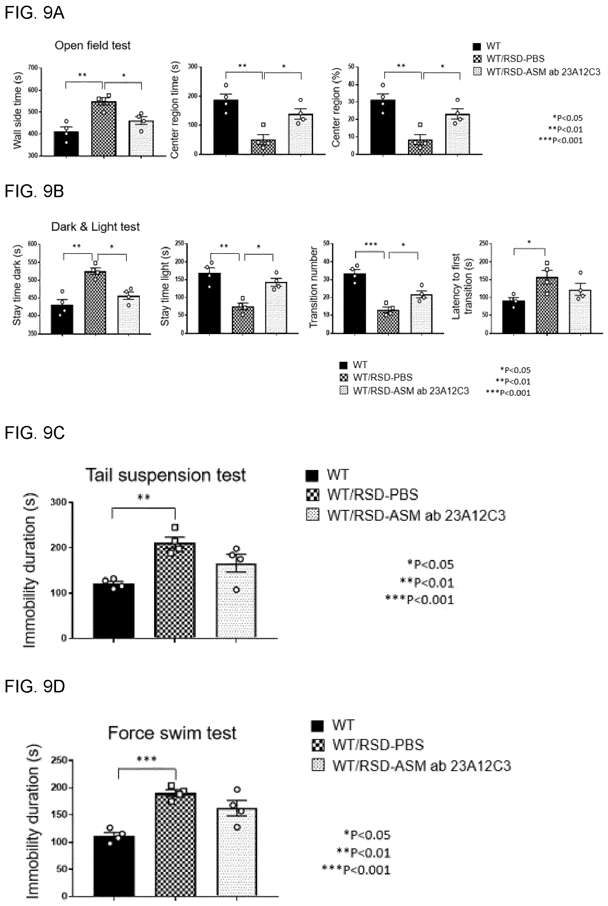

FIGS. 9A to 9D show results on the degree of recovery of depression behavior patterns in depression animal models injected with PBS or ASM-ab 23A12C3 through open field test (FIG. 9A), dark & light test (FIG. 9B), tail suspension test (FIG. 9C), and force swim test (FIG. 9D) (n=4/group) (WT: wild type, WT/RSD: depression-induced mouse model).

### MODE FOR INVENTION

Hereinafter, the present invention will be described in detail by the following embodiments. However, the following embodiments are only for illustrating the present invention, and the present invention is not limited thereto.

### Experimental Method

### 1. Production of ASM antibody

A monoclonal ASM antibody 23A12C3 which can inhibit ASM activities was produced by Koma biotech in order to verify the therapeutic effect of inhibiting of ASM activities in animal models with Alzheimer's, Lou Gehrig's, and depression. The sequences of the antibodies are shown in Tables 1 and 2 below.

**[Table 1]**

| **23A12C3 Light chain and Heavy chain CDR (complementarity determining region) sequence (5'→3')** | | |
|---|---|---|
| VH (Heavy chain variable | CDR1 | (SEQ IN NO: 1) DYYMN |
| | CDR2 | (SEQ IN NO: 2) VINPYNDGTSYNQKFKG |
| region) Amino acid sequence | CDR3 | (SEQ IN NO: 3) EKLYYYGRDYAMDY |
| VL (Light chain variable region) Amino acid sequence | CDR1 | (SEQ IN NO:4) RSSQDISNYLN |
| | CDR2 | (SEQ IN NO: 5) YTSRLHS |
| | CDR3 | (SEQ IN NO: 6) QQDNTLPYT |
| VH (Heavy chain variable region) DNA sequence | CDR1 | (SEQ IN NO: 7) GACTACTATATGAAC |
| | CDR2 | (SEQ IN NO: 8) GTTATTAATCCTTACAACGATGGTACTAGC TACAACCAGAAGTTCAAGGGC |
| | CDR3 | (SEQ IN NO: 9) GAGAAGCTTTATTACTACGGTAGGGACTAT GCTATGGACTAC |
| VL (Light chain variable region) DNA sequence | CDR1 | (SEQ IN NO: 10) AGGTCAAGTCAGGACATTAGCAATTATTTA AAC |
| | CDR2 | (SEQ IN NO: 11) TACACATCAAGATTACACTCA |
| | CDR3 | (SEQ IN NO: 12) CAACAGGATAATACGCTTCCGTACACG |

**[Table 2]**

| | |
|---|---|
| **23A12C3 antibody Heavy chain and Light chain sequence (5'→3')** | |
| Heavy chain (HC) | **FR1**-CDR1-**FR2**-CDR2-**FR3**-CDR3-**FR4** (SEQ IN NO:    13) EVQLQQSGPVLVKPGASVKMSCKASGYTFTDYYMNWVK QSHGKSLEWIGVINPYNDGTSYNQKFKGKATLTVDKSSST AYMELNSLTSEDSAVYYCAREKLYYYGRDYAMDYWGQG TSVTVSS |
| Amino acid sequence (466 aa) | |
| | Signal sequence-**FR1**-CDR1-**FR2**-CDR2-**FR3**-CDR3-**FR4-***Constant region*-Stop codon (SEQ IN NO: 14) MGWSWIFLFLLSGTAGVHSEVQLQQSGPVLVKPGASVKM SCKASGYTFTDYYMNWVKQSHGKSLEWIGVINPYNDGTS YNQKFKGKATLTVDKSSSTAYMELNSLTSEDSAVYYCAR EKLYYYGRDYAMDYWGQGTSVTVSSAKTTPPSVYPLAPG SAAQTNSMVTLGCLVKGYFPEPVTVTWNSGSLSSGVHTFP AVLQSDLYTLSSSVTVPSSTWPSQTVTCNVAHPASSTKVD KKIVPRDCGCKPCICTVPEVSSVFIFPPKPKDVLTITLTPKVT |
| | CVVVDISKDDPEVQFSWFVDDVEVHTAQTKPREEQINSTF RSVSELPIMHQDWLNGKEFKCRVNSAAFPAPIEKTISKTKG RPKAPQVYTIPPPKEQMAKDKVSLTCMITNFFPEDITVEWQ WNGQPAENYKNTQPIMDTDGSYFVYSKLNVQKSNWEAGN TFTCSVLHEGLHNHHTEKSLSHSPGK |
| Light chain (LC) | **FR1**-CDR1-**FR2**-CDR2-**FR3**-CDR3-**FR4** |
| Amino acid sequence (233 aa) | (SEQ IN NO: 15) DIQMTQTTSSLSASLGDRVTISCRSSQDISNYLNWCQQKP DGTVKLLIYYTSRLHSGVPSRFSGSGSGTDYSLTISNLEQE DIATYFCQQDNTLPYTFGGGTKLEIK |
| | Signal sequence-**FR1-**CDR1-**FR2**-CDR2-**FR3**-CDR3-**FR4**-*C onstant region*-Stop codon (SEQ IN NO: 16) MSSAQFLGLLLLCFQGTRCDIQMTQTTSSLSASLGDRVTIS CRSSQDISNYLNWCQQKPDGTVKLLIYYTSRLHSGVPSRF SGSGSGTDYSLTISNLEQEDIATYFCQQDNTLPYTFGGGT KLEIKRADAAPTVSIFPPSSEQLTSGGASVVCFLNNFYPKDI NVKWKIDGSERQNGVLNSWTDQDSKDSTYSMSSTLTLTK DEYERHNSYTCEATHKTSTSPIVKSFNRNEC |
| Heavy chain (HC) | **FR1**-CDR1-**FR2**-CDR2-**FR3**-CDR3-**FR4** (SEQ IN NO: 17) GAGGTCCAGCTGCAACAGTCTGGACCTGTGCTGGTGA AGCCTGGGGCTTCAGTGAAGATGTCCTGTAAGGCTTCT GGATACACATTCACTGACTACTATATGAACTGGGTGAAA CAGAGCCATGGAAAGAGCCTTGAGTGGATTGGAGTTAT TAATCCTTACAACGATGGTACTAGCTACAACCAGAAGTT CAAGGGCAAGGCCACATTGACTGTTGACAAGTCCTCCA GCACAGCCTACATGGAGCTCAACAGCCTGACATCTGA GGACTCTGCAGTCTATTACTGTGCAAGAGAGAAGCTTT ATTACTACGGTAGGGACTATGCTATGGACTACTGGGGTC AAGGAACCTCAGTCACCGTCTCCTCA |
| DNA sequence (1401 bp) | |
| | Signal sequence-**FR1**-CDR1-**FR2**-CDR2-**FR3**-CDR3-**FR4-***Constant region*-Stop codon (SEQ IN NO:    18) ATGGGATGGAGCTGGATCTTTCTCTTCCTCCTGTCAGGA ACTGCAGGTGTCCACTCTGAGGTCCAGCTGCAACAGTC TGGACCTGTGCTGGTGAAGCCTGGGGCTTCAGTGAAG ATGTCCTGTAAGGCTTCTGGATACACATTCACTGACTAC TATATGAACTGGGTGAAACAGAGCCATGGAAAGAGCCT TGAGTGGATTGGAGTTATTAATCCTTACAACGATGGTAC TAGCTACAACCAGAAGTTCAAGGGCAAGGCCACATTGA |
| | CTGTTGACAAGTCCTCCAGCACAGCCTACATGGAGCTC AACAGCCTGACATCTGAGGACTCTGCAGTCTATTACTG TGCAAGAGAGAAGCTTTATTACTACGGTAGGGACTATGC TATGGACTACTGGGGTCAAGGAACCTCAGTCACCGTCT CCTCAGCCAAAACGACACCCCCATCTGTCTATCCACTGG CCCCTGGATCTGCTGCCCAAACTAACTCCATGGTGACCC TGGGATGCCTGGTCAAGGGCTATTTCCCTGAGCCAGTG ACAGTGACCTGGAACTCTGGATCCCTGTCCAGCGGTGT GCACACCTTCCCAGCTGTCCTGCAGTCTGACCTCTACAC TCTGAGCAGCTCAGTGACTGTCCCCTCCAGCACCTGGC CCAGCCAGACCGTCACCTGCAACGTTGCCCACCCGGCC AGCAGCACCAAGGTGGACAAGAAAATTGTGCCCAGGGA TTGTGGTTGTAAGCCTTGCATATGTACAGTCCCAGAAGT ATCATCTGTCTTCATCTTCCCCCCAAAGCCCAAGGATGT GCTCACCATTACTCTGACTCCTAAGGTCACGTGTGTTGT GGTAGACATCAGCAAGGATGATCCCGAGGTCCAGTTCA GCTGGTTTGTAGATGATGTGGAGGTGCACACAGCTCAG ACGAAACCCCGGGAGGAGCAGATCAACAGCACTTTCCG TTCAGTCAGTGAACTTCCCATCATGCACCAGGACTGGCT CAATGGCAAGGAGTTCAAATGCAGGGTCAACAGTGCAG CTTTCCCTGCCCCCATCGAGAAAACCATCTCCAAAACCA AAGGCAGACCGAAGGCTCCACAGGTGTACACCATTCCA CCTCCCAAGGAGCAGATGGCCAAGGATAAAGTCAGTCT GACCTGCATGATAACAAACTTCTTCCCTGAAGACATTACT GTGGAGTGGCAGTGGAATGGGCAGCCAGCGGAGAACT ACAAGAACACTCAGCCCATCATGGACACAGATGGCTCTT ACTTCGTCTACAGCAAGCTCAATGTGCAGAAGAGCAACT GGGAGGCAGGAAATACTTTCACCTGCTCTGTGTTACATG AGGGCCTGCACAACCACCATACTGAGAAGAGCCTCTCC CACTCTCCTGGTAAATGA |
| Light chain (LC) | **FR1**-CDR1-**FR2**-CDR2-**FR3**-CDR3-**FR4** (SEQ IN NO: 19) GATATCCAGATGACACAGACTACATCCTCCCTGTCTGC CTCTCTGGGAGACAGAGTCACCATCAGTTGCAGGTCAA GTCAGGACATTAGCAATTATTTAAACTGGTGTCAGCAGA AACCAGATGGAACTGTTAAACTCCTGATCTACTACACAT CAAGATTACACTCAGGAGTCCCATCAAGATTCAGTGGC AGTGGGTCTGGAACAGATTATTCTCTCACCATTAGCAA CCTGGAGCAAGAAGATATTGCCACTTACTTTTGCCAAC AGGATAATACGCTTCCGTACACGTTCGGAGGGGGGACC AAGCTGGAAATAAAA |
| DNA sequence (702 bp) | |
| | Signal sequence-**FR1**-CDR1-**FR2**-CDR2-**FR3**-CDR3-**FR4**- |
| | *Constant region*-Stop codon (SEQ IN NO:    20) ATGTCCTCTGCTCAGTTCCTTGGTCTCCTGTTGCTCTGTT TTCAAGGTACCAGATGTGATATCCAGATGACACAGACT ACATCCTCCCTGTCTGCCTCTCTGGGAGACAGAGTCAC CATCAGTTGCAGGTCAAGTCAGGACATTAGCAATTATTT AAACTGGTGTCAGCAGAAACCAGATGGAACTGTTAAAC TCCTGATCTACTACACATCAAGATTACACTCAGGAGTCC CATCAAGATTCAGTGGCAGTGGGTCTGGAACAGATTAT TCTCTCACCATTAGCAACCTGGAGCAAGAAGATATTGC CACTTACTTTTGCCAACAGGATAATACGCTTCCGTACAC GTTCGGAGGGGGGACCAAGCTGGAAATAAAACGGGCT GATGCTGCACCAACTGTATCCATCTTCCCACCATCCAGT GAGCAGTTAACATCTGGAGGTGCCTCAGTCGTGTGCTTC TTGAACAACTTCTACCCCAAAGACATCAATGTCAAGTGG AAGATTGATGGCAGTGAACGACAAAATGGCGTCCTGAAC AGTTGGACTGATCAGGACAGCAAAGACAGCACCTACAG CATGAGCAGCACCCTCACGTTGACCAAGGACGAGTATG AACGACATAACAGCTATACCTGTGAGGCCACTCACAAGA CATCAACTTCACCCATTGTCAAGAGCTTCAACAGGAATG AGTGTTAG |

### 2. Mouse

Approval for mouse experiments was obtained from the Kyungpook National University Institutional Animal Care and Use Committee (IACUC). Based on C57BL/6 mice (Charles River, UK), transgenic mouse lines overexpressing APPswe (hAPP695swe) or PS1 (presenilin-1M146V) were used (hereinafter, APP mouse: mouse overexpressing APPswe, PS1 mouse: mouse overexpressing presenilin-1M146V; GlaxoSmithKline). In order to confirm the therapeutic effect of ASM activity inhibition (ASM antibody), 7-month-old APP/PS1 mice were injected intraperitoneally with PBS or ASM antibody 23A12C3 at a dose of 50 mg/kg twice a week. One month after injection of PBS or ASM antibody 23A12C3, behavioral analysis was performed, and brain tissues of mice were sampled after the behavioral analysis (FIG. 1A).

As an ALS animal model, the FUS-R521C transgenic mice were generated using Syrian hamster prion promoter driving the expression of FLAG-tagged human FUS-R521C cDNA. In order to confirm the therapeutic effect of ASM activity inhibition (ASM antibody 23A12C3) in ALS animal models, 7-week-old ALS mice were injected intraperitoneally with PBS or ASM antibody 23A12C3 at a dose of 50 mg/kg twice a week. Four weeks after injection of PBS or ASM antibody 23A12C3, behavioral analysis was performed, and mouse serum was sampled after the behavioral analysis (FIG. 6A).

For the depression-induced animal models, Repeated Social Defeat (RSD) stress was induced in C57BL/6 mice (Charles River, UK) for 10 days to be used as mice with depression. In order to induce Repeated Social Defeat (RSD) stress, two 6-8 week old male C57BL/6 mice were placed with one 6-8 week old male CD-1 mouse (aggressive intruder mouse) in the same cage for 2 hours daily for 10 days. After 10 days, the induction of depression in C57BL/6 mice was confirmed through a depression behavior test. In order to confirm the therapeutic effect of ASM activity inhibition (ASM antibody 23A12C3) in animal models with depression, 6-week-old male C57BL/6 mice were injected intraperitoneally with PBS or ASM antibody 23A12C3 at a dose of 50 mg/kg twice a week for 4 weeks before depression induction. Four weeks later, Repeated Social Defeat (RSD) stress was induced for 10 days, and behavioral analysis was performed. After the behavioral analysis, mouse serum was sampled (FIG. 8A).

### 3. Measurement of ASM activities

The concentration level of ASM was measured as follows. Specifically, 3 µl of mouse serum samples were mixed with ASM activity buffer and stored at 37°C. 114 µl of ethanol was added thereto to terminate the hydrolysis reaction, and centrifugation was performed. 30 µl of supernatant was transferred to a glass vial, and 5 µl was applied to the UPLC system. The concentration level of ASM was quantified by comparing same with Bodipy (aminoacetaldehyde) bound to sphingomyelin and ceramide. The extraction and quantification of the sphingomyelin and ceramides were performed by extracting lipids from samples by known methods, and resuspending the dried lipid extract in 25 µl of 0.2% Igepal CA-630 (Sigma-Aldrich), and quantifying the concentration level of each lipid by using the UPLC system.

### 4. Measurement of concentration of ASM proteins

ASM ELSIA (Mybiosource, MBS724194) was used to measure the concentration of ASM proteins in the serums of mice.

### 5. Immunofluorescence

The cerebrum and hippocampus of mice were fixed, and 0.5% thioflavin S (Sigma-Aldrich), anti-GFAP (rabbit, 1:500, DAKO), and anti-Iba-1 (rabbit, 1:500, WAKO) were cultured therein together. The portions were analyzed by using a laser scanning confocal microscope equipped with Fluoview SV1000 imaging software (Olympus FV1000, Japan), or an Olympus BX51 microscope. The percentage of area of the stained portions relative to the area of the total tissue was quantified and analyzed by using Metamorph software (Molecular Devices).

### 6. Western blot

The expression of the following genes was analyzed by using Western blotting. First, antibodies against LC3, p62 [all purchased from Cell Signaling Technologies], Lamp1 (abcam), TFEB (Invitrogen), and β-actin (Santa Cruz) were used. Densitometric quantification was performed by using ImageJ software (US National Institutes of Health).

### 7. Abeta40 and Abeta42 ELISA

Proteins were extracted from the cerebrum and hippocampus of mice, and the amounts of Abeta40 (Invitrogen, KHB3481) and Abeta42 (Invitrogen, KHB3441) were measured by using ELISA.

### 8. Behavioral experiment

In order to determine potential effects on learning and memory, Morris water maze (MWM) test was performed. The MWM taught mice the task 4 times a day for 10 days, the platform was removed on Day 11, and a probe trial was performed.

For fear conditioning, mice were placed in a conditioning chamber, and sound stimulation (10 kHz, 70 dB) and electrical stimulation (0.3 mA, 1 s) were provided on the first day. Their memory for space was checked without stimulation in the same conditioning chamber as the first day on the second day. A fear memory test on which only sound stimulation was provided was performed in a different conditioning chamber on the third day.

In order to confirm the improvement in exercise capacity of the ALS animal models, Tail suspension test, Hanging wire test, and Rotarod test were performed sequentially at daily intervals. In order to check whether depression was improved, Open field test, Dark & Light test, Tail suspension test, and Force swim test were performed sequentially at daily intervals.

### 9. Statistical analysis

For comparison between two groups, student's T-test was performed, while, for comparison between multiple groups, repeated measures analysis of Tukey's HSD test and variance test were performed according to the SAS statistical package (release 9.1; SAS Institute Inc., Cary, NC). *p<0.05, **p<0.01, ***p<0.001 were considered significant.

### Experiment results

### 1. Confirmation of changes in ASM activities in Alzheimer's animal models administered with ASM antibody

In order to verify in vivo the effect of alleviating Alzheimer's lesions by inhibiting ASM activities, monoclonal anti-ASM antibody (ASM-ab 23A12C3) was administered intraperitoneally twice a week to experimental animal models of Alzheimer's disease (AD: APP/PS1 mouse) (FIG. 1A).

In order to check whether ASM activities were inhibited, plasma was first extracted from Alzheimer's animal models injected with PBS or ASM-ab 23A12C3 to confirm the ASM activities. As a result, it was confirmed that the concentration level of ASM was significantly low in the plasma of Alzheimer's animal models injected with ASM-ab 23A12C3 (FIG. 1B). On the other hand, the concentration of the ASM protein in plasma did not differ between groups (FIG. 1C). In other words, it was found that administration of ASM-ab 23A12C3 may inhibit the activities of ASM protein without affecting the concentration of ASM proteins in the plasma of Alzheimer's animal models.

### 2. Confirmation of amyloid-β deposition in Alzheimer's animal models administered with ASM antibody

In order to confirm whether inhibition of ASM activities by ASM-ab 23A12C3 injection has an effect on Alzheimer's lesions, the cortex and hippocampus of mice were first stained with thioflavin S (ThioS) according to a known method to confirm fibrillar amyloid-β deposition. In addition, ELSIA was performed on Aβ40 and Aβ42 to confirm amyloid-β deposition. As a result of the experiments, it was confirmed that fibrillar Aβ deposition (FIGS. 2A and 2B) and Aβ40 and Aβ42 depositions (FIGS. 3A and 3B) in the cortex and hippocampus of the Alzheimer's animal models injected with ASM-ab 23A12C3 were significantly low, as compared to those of the Alzheimer's animal models injected with PBS.

### 3. Confirmation of changes in neuroinflammation in Alzheimer's animal models administered anti-ASM antibodies

In order to confirm the effect of inhibition of ASM activities by ASM-ab 23A12C3 injection on changes in neuroinflammation in Alzheimer's animal models, the present inventors observed changes in microglia and astrocytes in the brains. It was confirmed that the activities of microglia (lba-1) and astrocytes (GFAP) in the cortex and hippocampus of the Alzheimer's animal models administered ASM-ab 23A12C3 were significantly reduced, as compared to those of the Alzheimer's animal models injected with PBS (FIGS. 4A and 4B). Therefore, it was confirmed that inhibition of ASM activities by ASM-ab 23A12C3 injection regulates neuroinflammatory responses in the Alzheimer's brain environment.

### 4. Confirmation of improvement in memory in Alzheimer's animal models administered anti-ASM antibody

In order to determine whether inhibition of ASM activities by ASM-ab 23A12C3 injection has a potential effect on memory in Alzheimer's animal models, Morris water maze (MWM) test and Fear conditioning test were performed.

As shown in FIGS. 5A to 5D, it was confirmed that Alzheimer's animal models injected with PBS showed severe impairments in spatial memory, cognition, and memory formation, but such impairments improved in Alzheimer's animal models injected with ASM-ab.

### 5. Confirmation of changes in ASM activities in ALS animal models administered anti-ASM antibody

In order to verify in vivo the effect of alleviating ALS lesions by inhibiting ASM activities, anti-ASM antibody (ASM-ab 23A12C3) was administered intraperitoneally twice a week to experimental animal models of ALS (FUS R512C mice) (FIG. 6A).

In order to check whether ASM activities were inhibited, plasma was first extracted from ALS animal models injected with PBS or ASM-ab 23A12C3 to confirm the ASM activities. As a result, it was confirmed that the concentration level of ASM activities was significantly low in the plasma of ALS animal models injected with ASM-ab 23A12C3 (FIG. 6B).

### 6. Confirmation of improvement in exercise capacity in ALS animal models administered anti-ASM antibody

In order to determine whether inhibition of ASM activities by ASM-ab 23A12C3 injection has a potential effect on exercise capacity in animal models, Tail suspension test, Hanging wire test, and Rotarod test were performed.

As shown in FIGS. 7A to 7C, it was confirmed that ALS animal models injected with PBS showed severe impairments in motor function, but such impairments improved in the ALS animal models administered ASM-ab 23A12C3.

### 7. Confirmation of changes in ASM activities in depression-induced animal models administered anti-ASM antibody

In order to verify in vivo the effect of alleviating depression lesions by inhibiting ASM activities, ASM antibody (ASM-ab 23A12C3) was administered intraperitoneally to experimental animal models of depression (RSD stress-induced mice; WT/RSD) twice a week (FIG. 8A).

In order to check whether ASM activities were inhibited, plasma was extracted from depression-induced animal models injected with PBS or ASM-ab 23A12C3 to confirm the ASM activities. As a result, it was confirmed that the level of ASM activities was significantly low in the plasma of depression-induced animal models injected with ASM-ab 23A12C3 (FIG. 8B).

### 8. Confirmation of improvement of depression in depression-induced animal models administered anti-ASM antibody

In order to confirm whether inhibition of ASM activities by ASM-ab 23A12C3 injection was effective in improving depression in depression-induced animal models, Open field test, Dark & Light test, Tail suspension test, and Force swim test were performed.

As shown in FIGS. 9A to 9D, it was confirmed that depression-induced animal models injected with PBS showed severe depression symptoms, but depression animal models administered ASM-ab 23A12C3 showed significant improvement in depression symptoms.

Considering the above-described results, the monoclonal anti-ASM antibody according to the present invention can inhibit ASM activities. In particular, it was confirmed that inhibition of ASM activities by injection of anti-ASM antibodies in Alzheimer's animal models, ALS animal models, and depression-induced animal models can reduce Aβ plaque deposition and inflammatory response and improve learning and memory ability in Alzheimer's animal models, and improve exercise capacity and improve depression symptoms in ALS animal models and depression-induced animal models.

### INDUSTRIAL APPLICABILITY

The ASM antibody according to the present invention has very high sensitivity and specificity for ASM to be used to diagnose neurodegenerative diseases through detection of ASM, and is also very effective in inhibiting the activities of ASM to be used in the development of preventive or therapeutic agents for various neurodegenerative diseases such as Alzheimer's disease, and depression. Therefore, the present invention has a high industrial applicability.

## Claims

1. An antibody or antigen-binding fragment thereof that specifically binds to acid sphingomyelinase (ASM), comprising a heavy chain variable region comprising heavy chain complementarity determining region (CDR) 1 comprising the amino acid sequence defined by SEQ ID NO: 1, heavy chain CDR2 comprising the amino acid sequence defined by SEQ ID NO: 2, and heavy chain CDR3 comprising the amino acid sequence defined by SEQ ID NO: 3; and a light chain variable region comprising light chain CDR1 comprising the amino acid sequence defined by SEQ ID NO: 4, light chain CDR2 comprising the amino acid sequence defined by SEQ ID NO: 5, and light chain CDR3 comprising the amino acid sequence defined by SEQ ID NO: 6.

2. The antibody or antigen-binding fragment thereof of claim 1, wherein the antibody or antigen-binding fragment thereof comprises a heavy chain variable region (VH) comprising the amino acid sequence defined by SEQ ID NO: 13, and a light chain variable region (VL) comprising the amino acid sequence defined by SEQ ID NO: 15.

3. The antibody or antigen-binding fragment thereof of claim 1, wherein the antibody is selected from the group consisting of IgG, IgA, IgM, IgE, and IgD.

4. The antibody or antigen-binding fragment thereof of claim 1, wherein the antigen-binding fragment of the antibody is selected from the group consisting of diabody, Fab, Fab', F(ab)2, F(ab')2, Fv, and scFv.

5. A polynucleotide encoding an antibody or antigen-binding fragment thereof of any one of claims 1 to 4.

6. A vector comprising the polynucleotide of claim 5.

7. A host cell transformed with the vector of claim 6.

8. A pharmaceutical composition for preventing or treating neurodegenerative diseases, comprising an antibody or antigen-binding fragment thereof of any one of claims 1 to 4 as an active ingredient.

9. The composition of claim 8, wherein the neurodegenerative disease is at least one type selected from the group consisting of Alzheimer's disease, Parkinson's disease, progressive supranuclear palsy, multiple system atrophy, olivopontocerebellar atrophy (OPCA), Shay-Drager syndrome, striato-substantia nigra degeneration, Huntington's disease, amyotrophic lateral sclerosis (ALS), essential tremor, cortico-basal degeneration, diffuse Lewy body disease, Parkinson-ALS-dementia complex, Niemann-Pick disease, Pick's disease, cerebral ischemia, and cerebral infarction.

10. A pharmaceutical composition for preventing or treating depression, comprising an antibody or antigen-binding fragment thereof of any one of claims 1 to 4 as an active ingredient.

11. A composition for diagnosing neurodegenerative diseases, comprising an antibody or antigen-binding fragment thereof of any one of claims 1 to 4.

12. Use of an antibody or antigen-binding fragment thereof of any one of claims 1 to 4 for preparing a composition for treating neurodegenerative diseases.

13. A method for treating neurodegenerative diseases, comprising administering to a subject in need thereof an effective amount of a composition comprising an antibody or antigen-binding fragment thereof of any one of claims 1 to 4 as an active ingredient.

14. Use of an antibody or antigen-binding fragment thereof of any one of claims 1 to 4 for preparing a composition for treating depression.

15. A method for treating depression, comprising administering to a subject in need thereof an effective amount of a composition comprising an antibody or antigen-binding fragment thereof of any one of claims 1 to 4 as an active ingredient.

16. A method for diagnosing neurodegenerative diseases, comprising:
(a) obtaining a sample from a subject to bind thereto an antibody that specifically binds to acid sphingomyelinase (ASM); and
(b) diagnosing a neurodegenerative disease based on the measured activities of ASM.
